# EUROPEAN PATENT APPLICATION

(11) **EP 2 450 038 A1**
(43) Date of publication of application: **09.05.2012**
(21) Application number: 09833902.1
(22) Date of filing: 31.03.2009
(51) Int. Cl.: A61K 31/37, A61K 9/08, A61K 9/16, A61K 9/20, A61K 9/48, A61P 3/10, A61P 13/12

(54) **A PHARMACEUTICAL COMPOSITION FOR TREATING DIABETIC NEPHROPATHY AND THE PREPARATION METHOD AND USE THEREOF**

(30) Priority: 16.01.2009 CN 200910036715
(71) Applicant: Guangzhou Consun Medicine R&D Co., Ltd., Guangzhou, Guangdong 510530 (CN)
(72) Inventor: ZHU, Quan, Guangzhou Guangdong 510530 (CN); SHI, Xinghua, Guangzhou Guangdong 510530 (CN); TANG, Dan, Guangzhou Guangdong 510530 (CN); ZHENG, Zhaoguang, Guangzhou Guangdong 510530 (CN); HE, Bao, Guangzhou Guangdong 510530 (CN); DUAN, Tingting, Guangzhou Guangdong 510530 (CN); GU, Fei, Guangzhou Guangdong 510530 (CN); CHENG, Huiquan, Guangzhou Guangdong 510530 (CN); HUANG, Xiaoling, Guangzhou Guangdong 510530 (CN); HUANG, Yanxia, Guangzhou Guangdong 510530 (CN); WANG, Rushang, Guangzhou Guangdong 510530 (CN)
(74) Representative: Tansini, Elio Fabrizio
(86) International application number: PCT/CN2009/000343
(87) International publication number: WO 2010/081262

(57) **Abstract**

The invention relates to a pharmaceutical composition for diabetic nephropathy and its preparation, and the application in preparing of medicine for diabetic nephropathy. The pharmaceutical composition is made up of at least one of 7-hydroxycoumarin and 7-hydroxy-6-methoxycoumarin as active ingredient, and the weight percentage of active ingredient is 0.1-99.5%. Moreover, the pharmaceutical composition is mainly used for the prevention and cure of diabetic nephropathy. For the abundant material, remarkable effect on prevention and cure for diabetic nephropathy and convenience for use, it will be a new facultative drug for patients.

## Description

### FIELD OF THE INVENTION

The present invention relates to the flied of preparation of traditional Chinese medicine, in particular to a pharmaceutical composition for diabetic nephropathy and its preparation and application.

### BACKGROUND OF THE INVENTION

Diabetes mellitus and its chronic complications have seriously affected the healthy and life quality of human being. The number of patient has increased in recent years and the attack rate of diabetic nephropathy in every age group is 2.8% in 2000 when in 2030 is predicted to be 4.4% all around the world, which means that the number of diabetic patient will increase from 1.71 hundred millions in 2000 to 3.33 hundred million in 2030. With the increasing attack rate every year, the quantities of patients in China has been second only India. Therefore, the prevention and cure of diabetic and its complications has been a very important topic these days. Diabetic nephropathy (DN), one of the important complications of diabetic, has been the first place of diseases which leads to the final stage of renal failure. As shown by the data of world health organization that about 30% of I type and 25%-40% II type diabetic patients would improve to be DN patients if the blood glucose was not controlled. Because of delitescence and difficult to discover of DN in earlier period, it will can not be reverse when the symptom of renal affection appears. The yearlong diabetic can destroy filtration function of renal and express as microamount albuminuria. With the extend of disease course, albuminuria will increase and the ability of renal for eliminating toxin in blood decays gradually, which leads to the end stage renal disease (ESRD) and the patients can only maintain their life by hematodialysis or kidney transplant. Thereby, it is badly need to deeply elucidate the mechanism of DN, rich and consummate the measures of prevention and cure.

Cortex Mori, the dry root cortex of *Morus alba* L., tastes sweet, frigidity and possesses the functions of removing heat from lung and relieving asthima, inducing diuresis to alleviate edema. Cortex Mori is usually used for dyspnea and cough due to lung-heat, oliguria due to dropsy and turg, puffiness of face muscle and skin. The recent pharmacology researches showed that the Cortex Mori possessed the activities of lowering blood sugar and blood pressure, diuresis, preventing cough and relieving asthima, anti-HIV, anti-tumor and so on. Since the active ingredients of 1-deoxynojirimycin and moran A for lowering blood sugar were isolated from Cortex Mori, more and more scholars are looking for new active ingredients of lowering blood sugar from Cortex Mori and next to improve a new drug for diabetic. The main active ingredients of Cortex Mori are flavone, alkaloid, polysaccharides, coumarin (umbelliferone, scopoletin) and so on. The researches have proved that flavone, alkaloid and polysaccharides all posses the effect of lowering blood sugar and alkaloid performs the best. Coumarin in Cortex Mori is usually used for diuresis, preventing cough and relieving asthima, since now no literature has reported the effect on diabetic nephropathy. According to the research reports by domestic and overseas, coumarin possesses the bioactivities of anti-HIV, anti-tumor, lowering blood pressure, anti-arhythmia, anti-osteoporosis, analgesia, relieving asthima, antibiosis and so on. So it will be a new application in medical field for coumarin to treat diabetic nephropathy.

### SUMMARY OF THE INVENTION

It is an object of the invention to provide a pharmaceutical composition for diabetic nephropathy and its preparation, wherein the active ingredients of the pharmaceutical composition is coumarin.

To achieve the above object, the technical solution is as follows:

A pharmaceutical composition for diabetic nephropathy, the pharmaceutical composition is made up of at least one of 7-hydroxycoumarin and 7-hydroxy-6-methoxycoumarin as active ingredient, and the conventional drug carrier; the active ingredient is in a percentage of 0.1- 99.5% by weight.

Preferably, the active ingredient is in a percentage of 5- 70% by weight.

Preferably, the pharmaceutical composition is made up of 7-hydroxycoumarin and 7-hydroxy-6-methoxycoumarin as active ingredients, and the conventional carriers; the weight ratio of 7-hydroxycoumarin to 7-hydroxy-6-methoxycoumarin is in a range from 1:4 to 4:1.

The conventional drug carrier used in the pharmaceutical composition of the present invention is the conventional drug carrier of medical field. For example, the diluent and excipient is water; filler is starch, sucrose, lactose and so on; the adhesive is cellulose derivate, alginate, gelatin or polyvinylpyrrolidone; the wetting agent is glycerine; the disintegrant is one of the agar, calcium carbonate or sodium bicarbonate; the absorption enhancer is quaternary ammonium compound; the surfactant is palmityl alcohol; the adsorption carrier is kaolin clay or soap clay; the lubricant is talc, calcium stearate, magnesium stearate or polyethylene glycol. In addition, other adjuvant such as flavor agent, sweetener can also be added to the composition.

By using normal methods of preparation, the pharmaceutical composition of the present invention can be prepared into the normal dosage form which mainly is oral preparation including pellet, capsule, drug granules, dripping pills, oral liquid and so on.

The dose of the pharmaceutical composition can be adjusted according the administration way and the severity degree of disease. Normally, the oral reference dose of clinic is 10-70mold.

It is a further object of the present invention to provide a method for preparing a pharmaceutical composition for diabetic nephropathy, comprising: mixing at least one of 7-hydroxycoumarin and 7-hydroxy-6-methoxycoumarin with the conventional drug carrier; and preparing according the specific preparation process to obtain the pharmaceutical composition..

The pharmaceutical composition can be prepared according the normal preparing method in the pharmaceutical field. For example, mix the active ingredients with multi-carrier, then prepare to the dosage form as desired.

The present invention also provides the application of 7-hydroxycoumarin and/or 7-hydroxy-6methoxycoumarin in preparing the pharmaceutical composition for diabetic nephropathy.

### ADVANTAGES OF THE INVENTION

1. The present invention first time proves that the coumarin isolated from Cortex Mori has the function of lowering the blood sugar, which means to provide a new drug for the patients of diabetic and complications of diabetic.
2. The pharmaceutical composition of the present invention is much more effective than the water extract of Cortex Mori for diabetic nephropathy. In addition, because of the definite active ingredient and the easy control of quality, it overcomes the disadvantages of the traditional Chinese medicine in uncertainty of active ingredient, bad control of quality, many toxic side effects and so on.
3. The material for the pharmaceutical composition is abundance, it's easy to elaborated to the oral dosage form such as dripping pills, powder preparation for infusion, pellet and convenient for use.
4. The pharmaceutical composition possesses noticeable effect on the prevention and cure for the damage of glomcrulus in diabetic. The pharmaceutical composition can remarkably prevent the rise of serum creatinine and lower the urinary volume and urine protein of rat in diabetic, which proves that the pharmaceutical composition takes a remarkable effect on the prevention and cure on diabetic nephropathy.

### BRIEF DESCRIPTION OF THE DRAWINGS

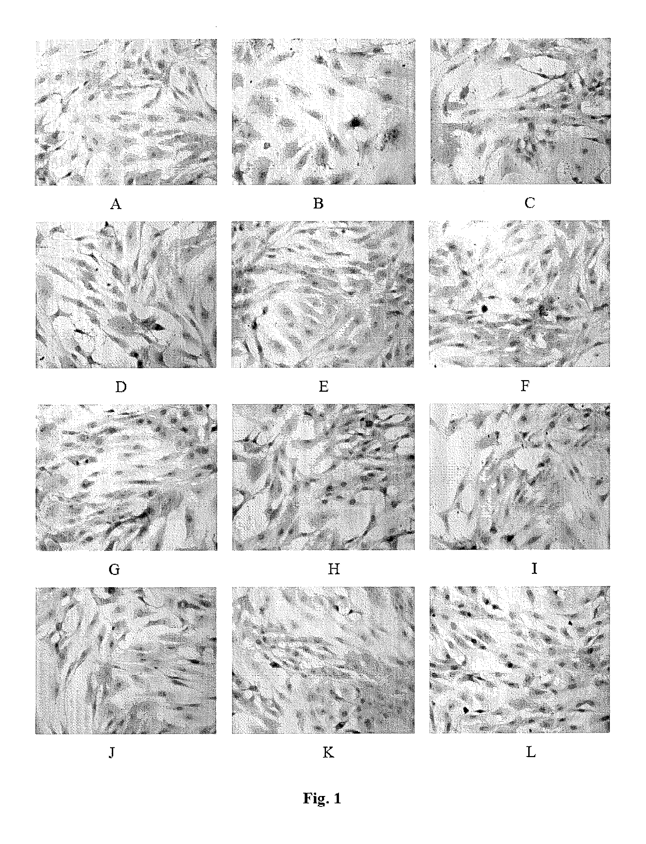

Fig.1 shows the changes on hypertrophy of rat glomcrulus intercapillary cells cultured under high glucose by 7-hydroxycouinarin and 7-hydroxy-6-methoxycoumarin (HE , 200x);
   wherein:
   A: normal control group;
   B: amylaceum (25mmol/L) group;
   C: water extract of Cortex Mori (0.1g/L);
   D: 7-hydroxycoumarin (1µmol/L);
   E: 7-hydroxycoumarin (0.1µmol/L);
   F: 7-hydroxycoumarin (0.01 µmol/L);
   G: 7-hydroxy-6-methoxycoumarin (1µmol/L);
   H: 7-hydroxy-6-methoxycoumarin (0.1µmol/L);
   I: 7-hydroxy-6-methoxycoumarin (0.01µmol/L);
   J: 7-hydroxycoumarin: 7-hydroxy-6-methoxycoumarin (0.06:0.03µmol/L);
   K: 7-hydroxycoumarin: 7-hydroxy-6-methoxycoumarin (0.05:0.05µmol/L);
   L: 7-hydroxycoumarin: 7-hydroxy-6-methoxycoumarin (0.03:0.06µmol/L).

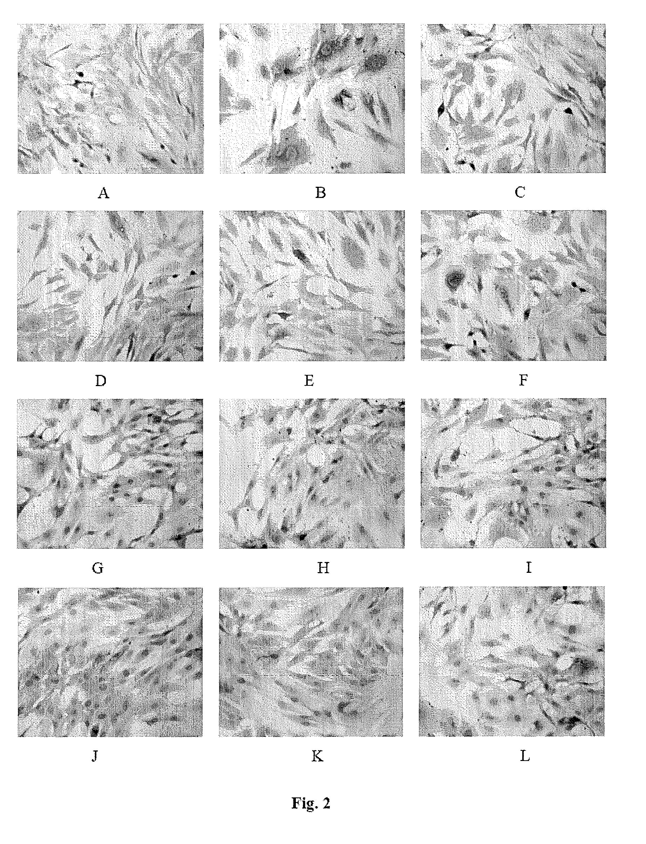

Fig. 2 shows the effect on extracellular matrix accrementition of rat glomcrulus intercapillary cells cultured under high glucose by 7-hydroxycoumarin and 7-hydroxy-6-methoxycoumarin (PAS , 200x);
   wherein:
   A: normal control group;
   B: amylaceum (25mmol/L) group;
   C: water extract of Cortex Mori (0.1g/L);
   D: 7-hydroxycoumarin (1µmol/L);
   E: 7-hydroxycoumarin (0.1µmol/L);
   F: 7-hydroxycoumarin (0.01µmol/L);
   G: 7-hydroxy-6-methoxycoumarin (1µmol/L);
   H: 7-hydroxy-6-methoxycoumarin (0.1µmol/L);
   I: 7-hydroxy-6-methoxycoumarin (0.01µmol/L);
   J: 7-hydroxycoumarin: 7-hydroxy-6-methoxycoumarin (0.06:0.03µmol/L);
   K 7-hydroxycoumarin: 7-hydroxy-6-methoxycoumarin (0.05: 0.05µmol/L);
   L: 7-hydroxycoumarin: 7-hydroxy-6-methoxycoumarin (0.03:0.06µmol/L).

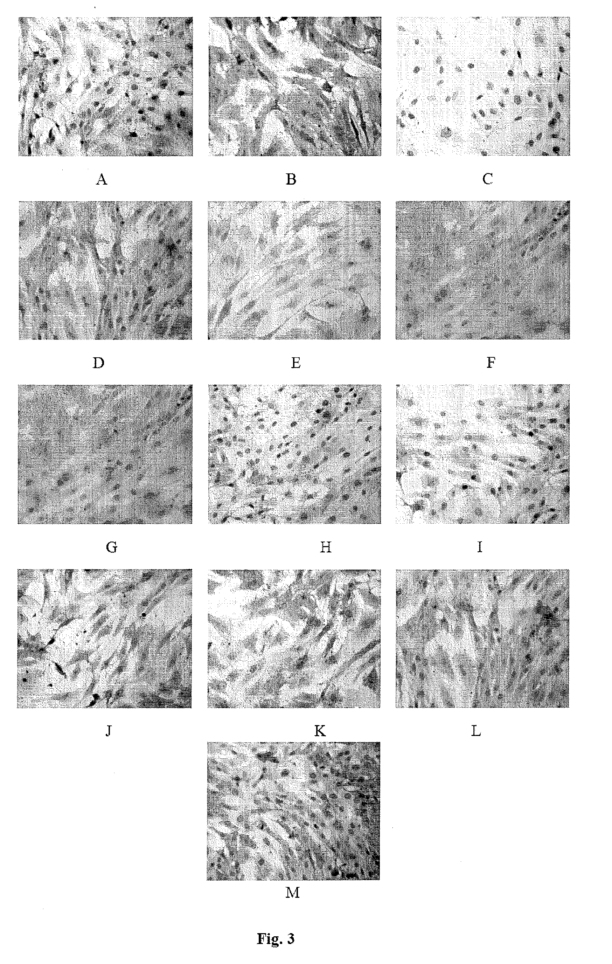

Fig. 3 shows the effect on CTGF express of rat glomcrulus intercapillary cells cultured under high glucose by 7-hydroxycoumarin and 7-hydroxy-6-methoxycoumarin (CTGF, 200× ).
   wherein:
   A: normal control group;
   B: amylaceum (25mmol/L) group;
   C: negative control (without CTGF antibody);
   D: water extract of Cortex Mori (0.1g/L);
   E: 7-hydroxycoumarin (1µmol/L);
   F: 7-hydroxycoumarin (0.1µmol/L);
   G: 7-hydroxycoumarin (0.01µmol/L);
   H: 7-hydroxy-6-methoxycoumarin (1µmol/L);
   I: 7-hydroxy-6-methoxycoumarin (0.1µmol/L);
   J: 7-hydroxy-6-methoxycoumarin (0.01µmol/L);
   K: 7-hydroxycoumarin: 7-hydroxy-6-methoxycoumarin (0.06:0.03µmol/L);
   L: 7-hydroxycoumarin: 7-hydroxy-6-methoxycoumarin (0.05:0.05µmol/L);
   M: 7-hydroxycoumarin: 7-hydroxy-6-methoxycoumarin (0.03:0.06µmol/L).

### DETAILED DESCRIPTION OF THE PREFERRED EMBODIMENT

The coumarin used in this invention for diabetic nephropathy is 7-hydroxycoumarin (A) and 7-hydroxy-6-methoxycoumarin (B), whose structures are shown as below:

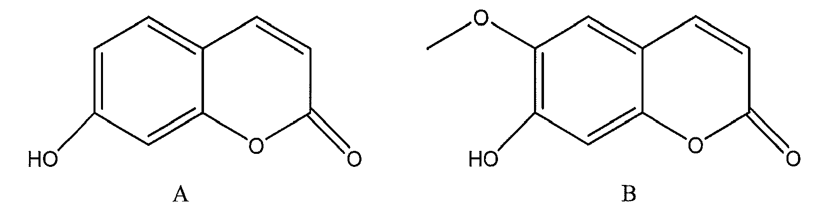

These two coumarin compounds are widely distribute in many plants and also can be attained by chemical synthesis.

The present invention will be further explained in the embodiments hereinafter.

### EXAMPLE 1: Preparation of 7-hydroxycoumarin

Triturate 10kg dry root cortex of Cortex Mori to fine power, add 100L water and extract under reflux for,2 hours thrice, combine the extract, concentrate to a certain volume under vacuum, add to the D101 macroporous resin, elute with water, then with 95% EtOH, collect the 95% EtOH elution and concentrate under vacuum, then add to the silica gel and elute with petroleum, petroleum-acetic ether 100:1, petroleum-acetic ether 50:1, petroleum-acetic ether 20:1 respectively. An amber power educes from the elution of petroleum-acetic ether 20:1, recrystallisation with acetone for several times provide the pure sample, the structure was identified as 7-hydroxycoumarin (umbelliferone) by infrared spectra, ultraviolet spectra, nuclear magnetic resonance and mass spectra. Molecular formula: C₉H₆O₃; molecular weight: 162. The purity detected by HPLC is >99% and the structure is shown as below:

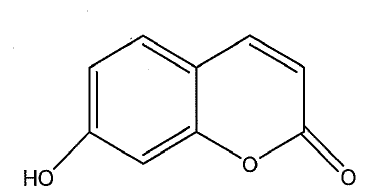

### EXAMPLE 2: Preparation of 7-hydroxy-6-methoycoumarin

Triturate 10kg dry root cortex of Cortex Mori to fine power, add 100L water and extract under reflux for 2 hours thrice, combine the extract, concentrate to a certain volume under vacuum, add to the D101 macroporous resin, elute with water, then with 95% EtOH, collect the 95% EtOH elution and concentrate under vacuum, then add to the silica gel and elute with petroleum, petroleum-acetic ether 100:1, petroleum-acetic ether 50:1 respectively. An amber power educes from the elution of petroleum-acetic ether 50:1, recrystallisation with acetone for several times provide the pure sample, the structure was identified as 7-hydroxy-6-methoxycoumarin (scopoletin) by infrared spectra, ultraviolet spectra, nuclear magnetic resonance and mass spectra. Molecular formula: C₁₀H₈O₄; molecular weight: 192. The purity detected by HPLC is >99% and the structure is shown as below:

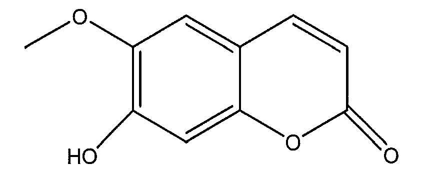

### EXAMPLE 3: Capsule preparation of the pharmaceutical composition (active ingredient: 7-hydroxy-6-methoxycoumarin)

### Steps:

A Take 4.2g beeswax, add 125g efamol, melt and mix under 80°Cwater bath, cool to the room temperature (25°C), then add sufficient quantity of butylated hydroxyarisol, mix uniformly.
B Add 0.25g 7-hydroxy-6-methoxycoumarin sieved with 100 mesh cribble and 5g lecithin, add 250g efamol, mix uniformly as the core liquid of capsule, then prepare into1000 capsules.

The capsule prepared in this example takes a satisfactory effect on hyperglycemia and diabetic nephropathy.

### EXAMPLE 4: Drop pill preparation of the pharmaceutical composition (active ingredient: 7-hydroxycoumarin)

Take 15g 7-hydroxycoumacin, triturate to fine power sieved with 200 mesh cribble, add to 15g melted polyglycol 6000 base material, agitate uniformly, prepare into drop pills with dimethyl benzene silicon oil as the chiller, dry and then finish. The drop pill takes a satisfactory effort on the disease for increase of serum creatinine and decrease of urine creatinine, and diabetic nephropathy.

### EXAMPLE 5: Pellet pill preparation of the pharmaceutical composition (active ingredient: 7-hydroxycoumarin or 7-hydroxy-6-methoxycoumarin)

The component per pill:

| | |
|---|---|
| 7-hydroxycoumarin or 7-hydroxy-6-methoxycoumarin | 12mg |
| Lactose | 175mg |
| Amylum maydis | 50mg |
| Magnesium stearate | 3mg |

Method of preparation: mix the active ingredient, lactose and starch, moisten uniformly with water, sieve the combination and dry, sieve again, add Magnesium stearate, then crush the combination into tablet with 240mg per pill and 12mg active ingredient. The pellet pill takes a satisfactory effort on disease of high urine protein and diabetic nephropathy.

### EXAMPLE 6: Dispersible tablet preparation of the pharmaceutical composition (active ingredient: 7-hydroxycoumarin and 7-hydroxy-6-methoxycoumarin)

Weight accurately a prescription dose of 95g 7-hydroxycoumarin, 190g 7-hydroxy-6-methoxy-coumarin, 40g lactose, 70g cellulose microcrystallisate, 5.25g polyvinylpolypyrrolidone, 2.5g aspartame, 6g polyvidone and 4g kalium vicarbonicum, misce bene the main medicine and the adjuvant by half-and-half increase, sieve with 80 mesh cribble for 2 times, put the combination to a proper container, add quantity sufficient of 15% (g/100m1) polyethylene 6000 solved in 75% EtOH, prepare soft material and make the wet granules by crushing through the 20 mesh cribble, spread on the vitreous enamel plaque, dry under 70°C for 2 hours in baking even of airblast, weigh accurately the dry granules, add 2.25g the remaining polyvinylpolypyrrolidone and 1% (w/w) sodium dodecyl sulphate(SDS), mix uniformly, crush into 1000 pills with φ7mm round chop-out die, then finish the 1000 dispersible tablets of 7-hydroxycoumarin and 7-hydroxy-6-methoxycoumarin. The dispersible tablet takes a satisfactory effort on diabetic nephropathy and its complications.

### EXAMPLE 7: Pellet pill preparation of the pharmaceutical composition (active ingredient: 7-hydroxycoumarin and 7-hydroxy-6-methoxycoumarin)

| | |
|---|---|
| Active ingredient: | 70g |
| Cellulose microcrystallisate: | 0.4g |
| | Crush into 1000pill |

Method of preparation: mix the active ingredients of 56g 7-hydroxycoumarin and 14g 7-hydroxy-6-methoxycoumarin with cellulose microcrystallisate, prepare into soft material by moistening uniformly with water, make the combination into granules, sieve with 20 mesh cribble, dry under 80°C, sieve again, then crush into pills of 70.4mg per pill and 70mg active ingredient per pill. The Pellet pill takes a satisfactory effort on diabetic nephropathy and its complications.

### EXAMPLE 8: Pellet pill preparation of the pharmaceutical composition (active ingredient: 7-hydroxycoumarin and 7-hydroxy-6-methoxycoumarin)

| | |
|---|---|
| Active ingredient: | 70g |
| Cellulose microcrystallisate: | 0.4g |
| | Crush into 1000pill |

Method of preparation: mix the active ingredient of 14g, 7-hydroxycoumarin and 56g 7-hydroxy-6-methoxycoumarin with cellulose microcrystallisate, prepare into soft material by moistening uniformly with water, make the combination into granules, sieve with 20 mesh cribble, dry under 80°C, sieve again, then crush into pills of 70.4mg per pill and 70mg active ingredient per pill. The Pellet pill takes a satisfactory effort on diabetic nephropathy and its complications.

In this invention, 7-hydroxycoumarin and/or 7-hydroxy-6-methoxycoumarin are applied to the experiments of effect on kidney intercapillary cells of rat cultured by high glucose and therapeutic action on db/db mouse of diabetic nephropathy.

### EXPERIMENT 1

Effects on hypertrophy of rat glomcrulus mesangial cells and extracellular matrix accrementition of rat glomcrulus intercapillary cells cultured under high glucose by 7-hydroxycoumarin and 7-hydroxy-6-methoxycoumarin.

The specific disease sign of diabetic nephropathy is glomerulosclerosis, and one of the important reasons is the overfull increase of extracellular matrix in mesangial region. Mesangial cell is the main component of glomcrulus mesenterium and takes an important role in mediating physiologic function of glomcrulus with many functions of contraction, endocrine secretion, division growth, immunity and phagocytosis. So the mesangial cell cultured under high glucose is a very valuable model for the research of glomerulosclerosis in diabetic nephropathy.

### 1. Material and agent

Rat messangial cell cell line HBZY-1; DMEM medium (GIBCO); newborn calf serum ( Sijiqing serum factory, Hangzhou); CTGF (Canta Cruz); biotinylation antibody of goat anti-mouse ( Jingmei bioengineering Co.,LTD); SABC staining kit of Immunohistochemistry for immediate use( Bost company); DAB coloration system (Gene Tech Biotechnology CompanyLimited).

CO₂ incubator (NAPC05410, PERCISION SCIENTIFIC); XSZ-D2 inverted microscope (Chongqing opticalinstrument factory); superclean bench (Baisheng technology network system Co., LTD, Suzhou & Purity technology institute, Suzhou city); Clinical centrifuge (LDZ5-2, Beijing clinical centrifuge factory); Immunohistochemical shi box (Maixin biotechnology explotitation company, Fuzhou).

### 2. Experimental method

### 2.1 Preparation of water extract of Cortex Mori

Triturate quantity sufficient dry root cortex of Cortex Mori to power, add 10 times water and extract under reflux for 3 times, 2 hours per time, combine the extract, concentrate to a certain volume under vacuum for use.

### 2.2 Modeling and culturing of cells

Take the rat mesangial cells in log phase growth, prepare into unicell suspension (5×10⁴ cells/ml) with 5% DMEM medium of newborn calf serum, inoculate to the 24 well plate coated with coverslip for 1ml/well(cell quantity: 5×10⁴/well), incubated under 5% CO₂ incubator at 37°C for 24 hours, added the DMEM medium without serum, incubated 24 hours to make all the cells to anestrum. Discard the supernatant, added 900µl DMEM medium and 100µl different concentration amylaceum (each concentration for 3 repeat) and incubated 24h, 48h, 72h and 96h, respectively. Observed the modeling results after staining with HE and PAS. The result shows that 25mmol/L amylaceum and incubating for 48h was the best condition for the model of the experiment.

Put the coverslips pre-treatment by poly-L-lysine into 24-well cell culture plates before added the unicell suspension, then cultured these cells until the step of synchronization. Discard of the supernatant, add 800µl DMEM medium, 100µl amylaceum(final concentration is 25mM/L) in each medicine group, then add 100µl water extract of Cortex Mori (final concentration is 0.1g/L or 7-hydroxycoumarin (final concentration is 1, 0.1, or 0.01µmol/L) or 7-hydroxy-6-methoxycoumarin (final concentration is 1, 0.1, or 0.01µmol/L) or mixed solution of 7-hydroxycoumarin and 7-hydroxy-6-methoxycoumarin (the final concentration is 0.06:0.03, 0.05:0.05, or 0.03:0.06 µmol/L). The normal control group is added 1 ml DEME medium when the high glucose group is added 900µl DMEM medium and 100µl amylaceum (final concentration was 25mmol/L). Each group repeats for 3 times. Then, all the groups are incubated under 5% CO₂ incubator at 37°C for 48h. After that, take out of the coverslips for HE staining, PAS staining and immunocytochemistry staining.

### 2.3 Staining

### Routine HE staining, routine PAS staining, CTGF staining.

Steps of CTGF dyeing: fix the coverslip coated with cell with 4% paraformaldehyde for 30min, deactivate the endogenous horse radish peroxidase for 10min with the 3% H₂O₂ solution of methanol prepared at that time, clean with distilled water for 3 times; block the non-specific antigen for 20 min with 5% BSA, add dropwise with antibody CTGF (:500), remain under 4°C for one night, wash for 20 min with PBS (PH 7.4) for 3 times, add dropwise biotinylation antibody of goat anti mouse, keep under 37 °Cfor 30 min, wash for 5 min with PBS (PH 7.4) for 4 times, add 50µl developer (add a drop of each developer A, B, C to 1ml distilled water and mix uniformly), color under room temperature for 15 min, clean uniformly with distilled water, dyeing lightly with hematoxylin again for 1 min, wash with water, dewater, transparent, cover the coverslip with neutro-gummi, observe the dyeing under microscope.

### 3 Result and analysis

Observe the hypertrophy change of rat mesangial cells, accrementition of extracellular matrix and express of CTGF, respectively.

### 3. 1 The changes on hypertrophy of rat glomcrulus intercapillary cells cultured under high glucose by 7-hydroxycoumarin and 7-hydroxy-6-methoxycoumarins

As shown in Fig. 1, the hypertrophy of rat mesangial cells cultured under high glucose (25mmol/L) is obvious, and water extract of Cortex Mori, mono-use or co-use of 7-hydrocomarin and 7-hydroxy-6-methoxycomarin all can restrain significantly the hypertrophy of rat mesangial cells induced by high glucose. Moreover, the effects of compounds are better than the extract and co-use is better or equal to mono-use.

### 3.2 Effect on extracellular matrix accrementition of rat mesangial cells cultured under high glucose by 7-hydroxycoumarin and 7-hydroxy-6-methoxycoumarin

As shown in Fig. 2, the extracellular matrix of rat mesangial cells cultured under high glucose (25mmol/L) for 48 hours increase ( the area and chromaticity of dyeing zone dyed by PAS increase), and water extract of Cortex Mori, mono-use or co-use of 7-hydrocomarin and 7-hydroxy-6-methoxycomarin all can decrease the extracellular matrix of rat mesangial cells induced by high glucose. Moreover, the effects of compounds are better than the extract and co-use is better than mono-use.

### 3.3 Effect on CTGF express of rat mesangial cells cultured under high glucose by 7-hydroxycoumarin and 7-hydroxy-6-methoxycoumarin

As shown in Fig. 3, the CTGF express of rat mesangial cells cultured under high glucose (25mmol/L) for 48 hours increase, and water extract of Cortex Mori, mono-use or co-use of 7-hydrocomarin and 7-hydroxy-6-methoxycomarin all can decrease the CTGF express of rat mesangial cells induced by high glucose. Moreover, the effects of compounds are better than the extract and co-use is better than mono-use.

### EXPERIMENT 2

Therapeutic action on db| db mouse of diabetic nephropathy by 7-hydrocomarin and 7-hydroxy-6-methoxycomarin

### 1 Material and agent

db| db mouse, B6 mouse (offered by experimental animal center of southern medical university); Accu-Chek Advantage blood sugar instrument (los diagnose company, America) ; SPECTRA max190 (America MD company); detection kit of creatinine, detection kit of Coomassie brilliant blue (Jiancheng bioengineering institute, Nangjing,China).

### 2 Method of experiment

### 2.1 Preparation of water extract of Cortex Mori

Triturate quantity sufficient dry root cortex of Cortex Mori to power, add 10 times water and extract under reflux for 3 times, 2 hours per time, combine the extract, concentrate to a certain volume under vacuum for use.

### 2.2 Animal management and administration

During the experiment,all the mouse are bred in lamina flow cage, free to food and water. Water and underpad are changed every day to keep the cage clean, and the water, seedstuff, underpad are stationary. Shine in alternation in 12 hour. All the appliances and food are sterilized everyday.

110 8-week-old mouse are divided into 11 groups with 10 mouse per group: model group, water extract of Cortex Mori (dose: 2000mg crude drug/kg), 7-hydroxycoumarin (dose: 0.5, 1.0 and 2.0mg/kg), 7-hydroxy-6-methoxycoumarin (dose: 0.5, 1.0 and 2.0mg/kg), 7-hydroxycoumarin & 7-hydroxy-6-methoxycoumarin (dose: 0.6:0.3, 0.5:0.5 and 0.3:0.6). Moreover, 10 8-week-old B6 mouse are taken as the normal control. Each group is given 0.4ml corresponding drug in different concertration or physiological saline by intragastric administration for 5 weeks.

### 3 Statistical analysis

Determinate the food-intake and water-intake, weigh every week,determinate the blood glucose every week. Collect the 24 hour urine after the administration is over to calculate the urine volume and determinate the total protein and creatinine of urine, sacrifice the mouse and receipt the double kidney to calculate the kidney index [RI:RI (‰)= kidney weight /body weight× 1000].

All the determination are statisticsed and analyzed by interclass *t*-test and expressed as x̅±s,

### 4 Results

### 4.1 Effect on the db| db mouse weight of diabetic nephropathy by 7-hydroxycoumarin and 7-hydroxy-6-methoxycoumarin

As shown in Table 1, water extract of Cortex Mori, mono-use or co-use of 7-hydrocomarin and 7-hydroxy-6-methoxycomarin all can restrain the weight of db| db mouse in diabetic nephropathy to increase (P<0.05). Moreover, the effects of 7-hydrocomarin and 7-hydroxy-6-methoxycomarin are better or equal than the extract, and co-use is better to mono-use.

**Table 1 Effect on the db| db mouse weight of diabetic nephropathy by 7-hydroxycoumarin and 7-hydroxy-6-methoxycoumarin (x±s, n=10)**

| | Animal quantity | Dosage (mg/kg) | Weight (g) | | | | | |
|---|---|---|---|---|---|---|---|---|
| | | | 0 week | 1 week | 2 weeks | 3 weeks | 4 weeks | 5 weeks |
| Normal control | 10 | - | 18.9±0.4 | 20.5±0.6* | 21.2±0.7** | 22.1±1.2** | 23.0±2.0** | 23.5±1.6** |
| model | 10 | - | 23.8±0.3 | 29.4±1.1 | 37.1±1.7 | 43.4±2.5 | 47.5±3.9 | 49.8±4.1 |
| Extract of MC | 10 | 2000 | 23.5±0.4 | 28.6±0.9 | 34.6±1.3* | 38.2±1.6* | 42.9±3.2 | 46.1±3.4* |
| | 10 | 0.5 | 24.5±0.5 | 31.5±1.5 | 35.8±1.8 | 39.4±2.0 | 43.9±3.1 | 46.4±4.6 |
| 7-hydroxy -coumarin | 10 | 1.0 | 23.4±0.5 | 30.2±1.4 | 34.6±1.4* | 40.0±2.7 | 43.1±2.8 | 45.2±4.1* |
| | 10 | 2.0 | 23.7±0.6 | 30.3±0.5 | 35.5±1.7 | 38.6±1.9* | 42.3±2.8* | 46.1±5.3 |
| | 10 | 0.5 | 23.2±0.6 | 30.6±1.2 | 36.4 ±1.5 | 40.1±2.5 | 43.0±2.1 | 47.0±3.9 |
| 7-hydroxy-6 -methoxycomarin | 10 | 1.0 | 24.1±0.5 | 31.8±1.0 | 33.7±1.4** | 38.8±1.4* | 42.5±2.5* | 45.8±3.0* |
| | 10 | 2.0 | 23.9±0.5 | 32.0±1.3 | 34.3±1.9* | 39.5±1.8 | 44.0±3.2 | 45.7±4.5* |
| 7-hydroxy -coumarin : | 10 | 0.6:0.3 | 24.3±0.6 | 31.3±1.0 | 36.1 ±1.4 | 38.9±2.0* | 42.8±2.2* | 46.2±3.6 |
| 7-hydroxy-6 | 10 | 0.5:0.5 | 23.1±0.4 | 31.0±1.4 | 34.5+1.6* | 38.2+1.2* | 43.1±2.4 | 45,1+3.3* |
| -methoxycomarin | 10 | 0.3:0.6 | 23.2±0.6 | 30.2±1.3 | 33.5±1.7** | 37.6±1.7* | 42.7±2.9* | 41.5±4.4** |

| | | | | | | | | |
|---|---|---|---|---|---|---|---|---|
| Vs model group, * P<0.05 , ** P<0.01 | | | | | | | | |

### 4.2 Effect on blood glucose of db| db mouse in diabetic nephropathy by 7-hydroxycoumarin and/or 7-hydroxy-6-methoxycoumarin

As shown in Table 2, mono-use or co-use of 7-hydrocomarin and 7-hydroxy-6-methoxycomarin at the final phase administration all can remarkably decrease the blood glucose of db| db mouse in diabetic nephropathy (P<0.05). Moreover, the effects of 7-hydrocomarin and 7-hydroxy-6-methoxycomarin are obviously better than the extract, and co-use is better to mono-use.

**Table 2 Effect on blood glucose of db| db mouse in diabetic nephropathy by 7-hydroxycoumarin and/or 7-hydroxy-6-methoxycoumarin (x±s, n=10)**

| | Animal quantity | Dosage (mg/kg) | Blood glucose value (mmol/L) | | | | |
|---|---|---|---|---|---|---|---|
| | | | 1week | 2 weeks | 3 weeks | 4 weeks | 5 weeks |
| Normal control | 10 | - | 7.0±1.5** | 6.9±1.1** | 6.6±0.7** | 7.1±0.9** | 4.4±1.2** |
| model | 10 | - | 13.7±2.8 | 13.5±3.0 | 12.4±3.6 | 14.1±4.3 | 13.2±3.1 |
| Extract of MC | 10 | 2000 | 13.9±3.4 | 14.0±3.1 | 12.8±2.5 | 10.9±2.8* | 11.3±3.8 |
| | 10 | 0.5 | 13.7±4.1 | 13.6±2.9 | 12.9±2.9 | 11.2±4.0 | 12.1±1.9 |
| 7-hydroxy -coumarin | 10 | 1.0 | 13.4±4.6 | 13.4±2.6 | 13.9±4.3 | 11.2±4.4 | 10.3±2.0* |
| | 10 | 2.0 | 13.0±3.6 | 13.1±5.1 | 13.7±5.1 | 10.1±2.4* | 10.6±3.0 |
| | 10 | 0.5 | 13.3±4.5 | 13.4±2.7 | 13.5±3.2 | 12.1±3.6 | 13.0±2.2 |
| 7-hydroxy-6 -methoxycomarin | 10 | 1.0 | 13.5±4.0 | 13.0±3.2 | 12.6±4.8 | 10.8±2.5* | 10.4±2.5* |
| | 10 | 2.0 | 13.8±4.7 | 13.3±2.7 | 13.1±4.7 | 10.4±3.9* | 11.3±4.4 |
| 7-hydroxy -coumarin : | 10 | 0.6:0.3 | 13.5±4.3 | 13.2±2.5 | 12.8±3.8 | 10.3±3.3* | 11.3±2.8 |
| | 10 | 0.5:0.5 | 13.6±4.8 | 13.3±2.9 | 12.1±2.7 | 11.5±2.7 | 10.1±2.2* |
| 7-hydroxy-6 -methoxycomarin | 10 | 0.3:0.6 | 13.2±4.1 | 13.0±2.4 | 11.5±4.5 | 10.2±3.3* | 9.9±3.7* |

| | | | | | | | |
|---|---|---|---|---|---|---|---|
| Vs model group, * P<0.05 , ** P<0.01 | | | | | | | |

### 4.3 Effect on urine volume, urine total protein and urine creatinine in 24 hours of db| db mouse in diabetic nephropathy by 7-hydroxycoumarin and/or 7-hydroxy-6-methoxycoumarin

As shown in Table 3, water extract of Cortex Mori, mono-use or co-use of 7-hydrocomarin and 7-hydroxy-6-methoxycomarin all can decrease the urine volume and urine total protein, and elevate the urine creatinine in urine in 24 hours of db| db mouse in diabetic nephropathy (P<0.05, P<0.01). Moreover, the effects of 7-hydroxy-6-methoxycomarin and 7-hydrocomarin are better than the extract, and co-use is better than mono-use.

**Table 3.Effect on urine volume, urine total protein and urine creatinine in 24 hours of db| db mouse in diabetic nephropathy by 7-hydroxycoumarin and/or 7-hydroxy-6-methoxycoumarin (x±s, n=10)**

| | Animal quantity | dosage (mg/kg) | Urine volume (ml) | Total protein (mg) | Urine creatinine (mmol/L) |
|---|---|---|---|---|---|
| Normal control | 10 | - | 0.12±0.06** | 0.135±0.07** | 12.4±2.59** |
| model | 10 | - | 1.18±0.40 | 1.614±0.64 | 1.56±0.50 |
| Extract of MC | 10 | 2000 | 0.84±0.34* | 1.126±0.468* | 2,23±0.57* |
| 7-hydroxy | 10 | 0.5 | 1.03±0.28 | 1.205±0.541 | 2.06±0.65 |
| -coumarin | 10 | 1.0 | 0.72±0.30* | 0.996±0.338* | 2.30±0.71* |
| | 10 | 2.0 | 0.74±0.33* | 1.147±0.305* | 2.79±0.54** |
| | 10 | 0.5 | 0.95±0.34 | 1.398±0.372 | 1.85±0.56 |
| 7-hydroxy-6-methoxycomarin | 10 | 1.0 | 0.75±0.25* | 1.102±0.249*^{#} | 3.20±0.85**^{#} |
| | 10 | 2.0 | 0.78±0.31* | 0.878±0.352**^{##} | 2.81±0.76** |
| 7-hydroxy -coumarin: | 10 | 0.6:0.3 | 0.90±0.35 | 1.163±0.335*^{#} | 2.35±0.64* |
| 7-hydroxy-6 | 10 | 0.5:0.5 | 0.79±0.25* | 1.109±0.318*^{#} | 3.34±0.79**^{#} |
| -methoxycomarin | 10 | 0.3:0.6 | 0.74±0.22* | 0.905±0.320*^{#} | 3.35±0.81**^{#} |

| | | | | | |
|---|---|---|---|---|---|
| Vs model group, * P<0.05, ** P<0.01; vs Extract ofMC, # p<0.05, ## p<0.01 | | | | | |

### 4.4 Effect on kidney weight and kidney index of db| db mouse in diabetic nephropathy by 7-hydroxycoumarin and/or 7-hydroxy-6-methoxycoumarin

As shown in Table 4, kidney weight and kidney index of db| db mouse in diabetic nephropathy increase after 5 weeks; water extract of Cortex Mori, mono-use or co-use of 7-hydrocomarin and 7-hydroxy-6-methoxycomarin all can obviously decrease kidney weight and kidney index of db| db mouse in diabetic nephropathy. Moreover, the effects of 7-hydroxy-6-methoxycomarin are better than the extract and 7-hydrocomarin, and co-use is better to mono-use.

**Table 4 Effect on kidney weight and kidney index of db| db mouse in diabetic nephropathy by 7-hydroxycoumarin and/or 7-hydroxy-6-methoxycoumarin (x±s, n=10)**

| | dosage (mg/kg) | Kidned weight(g) | Kidned index (‰) |
|---|---|---|---|
| Normal control | - | 0.26±0.04* | 7.0±1.2** |
| model | - | 0.30±0.04 | 13.1±1.7 |
| Extract of MC | 2000 | 0.26±0.04* | 11.2±1.5 |
| | 0.5 | 0.28±0.04 | 11.8±1.5 |
| 7-hydroxy -coumarin | 1.0 | 0.26±0.03* | 8.5.±0.8** |
| | 2.0 | 0.27±0.02* | 9.4±0.3* |
| | 0.5 | 0.29±0.02 | 12.6±1.0 |
| 7-hydroxy-6-methoxycomarin | 1.0 | 0.25±0.03* | 9.3.±1.2* |
| | 2.0 | 0.24±0.04* | 9.1±1.7* |
| 7-hydroxy-coumarin: | 0.6:0.3 | 0.25±0.03* | 10.7±1.5* |
| 7-hydroxy-6 | 0.5:0.5 | 0.26±0.05* | 9.9.±1.4* |
| -methoxycomarin | 0.3:0.6 | 0.24±0.03* | 9.1±1.7* |

| | | | |
|---|---|---|---|
| Vs model group, * P<0.05 , ** P<0.01 | | | |

## Claims

1. A pharmaceutical composition for diabetic nephropathy, **characterized in that**: the pharmaceutical composition is made up of at least one of 7-hydroxycoumarin and 7-hydroxy-6-methoxycoumarin as active ingredient, and the conventional drug carrier; the active ingredient is in a percentage of 0.1- 99.5% by weight.

2. The pharmaceutical composition of claim 1, **characterized in that** the active ingredient is in a percentage of 5- 70% by weight.

3. The pharmaceutical composition of claim 2, **characterized in that** the pharmaceutical composition is made up of 7-hydroxycoumarin and 7-hydroxy-6-methoxycoumarin as active ingredient, and the conventional drug carrier; the weight ratio of 7-hydroxycoumarin to 7-hydroxy-6-methoxycoumarin is in a range from 1:4 to 4:1.

4. The pharmaceutical composition of any one of claims 1 to 3, **characterized in that** the conventional drug carrier comprises at least one of diluent, excipient, filler, adhesive, wetting agent, disintegrant, absorption enhancer, surfactant, adsorption carrier, lubricant, flavor agent and sweetener, wherein the excipient is water ; the filter is starch, sucrose or lactose; the adhesive at least one selected from a group consisting of cellulose derivative, alginate, gelatin and polyvinylpyrrolidone; the wetting agent is glycerine; the disintegrant is agar, calcium carbonate or sodium bicarbonate; the absorption enhancer is quaternary ammonium compound ; the surfactant is palmityl alcohol; the adsorption carrier is kaolin clay or soap clay; the lubricant is talc, calcium stearate, magnesium stearate or polyethylene glycol.

5. The pharmaceutical composition of claim 4, **characterized in that** the pharmaceutical composition can be prepared into a clinical dosage form such as pellet, capsule, drug granules, dripping pills or oral liquid.

6. A method for preparing the pharmaceutical composition of claim 1, **characterized by** comprising: mixing at least one of 7-hydroxycoumarin and 7-hydroxy-6-methoxycoumarin with the conventional drug carrier; and preparing according the specific preparation process to obtain the pharmaceutical composition.

7. Use of 7-hydroxycoumarin and/or 7-hydroxy-6-methoxycoumarin in preparing a pharmaceutical composition for diabetic nephropathy.
